(19) Europäisches Patentamt
European Patent Office
Office européen des brevets

(11) Numéro de publication: **0 172 096**
**B1**

(12) **FASCICULE DE BREVET EUROPÉEN**

(45) Date de publication du fascicule du brevet: **25.01.89**

(51) Int. Cl.⁴: **C 07 D 471/00,** A 61 K 31/00

(21) Numéro de dépôt: **85401554.2**

(22) Date de dépôt: **30.07.85**

(54) **Dérivés d'acylaminométhyl-3 imidazo 1,2-a pyridines, leur préparation et leur application en thérapeutique.**

(30) Priorité: **07.08.84 FR 8412447**

(43) Date de publication de la demande: **19.02.86 Bulletin 86/8**

(45) Mention de la délivrance du brevet: **25.01.89 Bulletin 89/4**

(84) Etats contractants désignés: **AT BE CH DE FR GB IT LI LU NL SE**

(56) Documents cité: **GB-A-991 589**

(73) Titulaire: **SYNTHELABO, 58, rue de la Glacière, F-75621 Paris Cedex 13 (FR)**

(72) Inventeur: **George, Pascal, 39, rue Henri de Vilmorin, F-94400 Vitry S/Seine (FR)**
Inventeur: **Giron, Claudie, 1, Parvis de la Bièvre Apt. 76, F-92160 Antony (FR)**

(74) Mandataire: **Ludwig, Jacques, SYNTHELABO Service Brevets 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



LIBER, STOCKHOLM 1989

## Description

La présente invention a pour objet des dérivés d'acylaminométhyl-3 imidazo[1,2-a]pyridines, leur préparation et leur application en thérapeutique.

Les composés de l'invention répondent à la formule I donnée dans le schéma qui suit, formule dans laquelle X représente l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino,

Y représente l'hydrogène, le chlore ou un groupe méthyle,

$R^1$ représente l'hydrogène ou un groupe alkyle en $C_1$-$C_4$ linéaire ou ramifié, ou un groupe benzyle,

$R^2$ représente un groupe alkyle en $C_1$-$C_6$ linéaire ou ramifié, un groupe cyclohexyle, trichlorométhyle, propène-1 yle, allyle, phényle, chloro-4 phényle ou benzyle,

ou bien encore

$R^1$ et $R^2$ représentent ensemble une chaîne aliphatique en $C_3$-$C_5$.

Les composés de l'invention peuvent se présenter à l'état de bases libres ou de sels d'addition à des acides acceptables en pharmacologie.

Des dialkylaminométhyl-3 imidazo[1,2-a]pyridines sont décrites dans le brevet GB-991 589 comme ayant des propriétés anti-inflammatoires, antipyrétiques et analgésiques, alors que les composés de la présente invention sont actifs sur le système nerveux central, en particulier comme anxiolytiques et hypnotiques, comme on le verra dans la description des essais pharmacologiques donnée plus loin.

Les composés préféres sont ceux dans la formule I desquels X représente le chlore ou un groupe méthyle ou méthylthio, Y représente le chlore ou un groupe méthyle, $R^1$ représente l'hydrogène ou un groupe méthyle, et $R^2$ représente un groupe propyle ou isobutyle.

Les composés de l'invention peuvent être préparés conformément au schéma qui suit.

**Schéma**

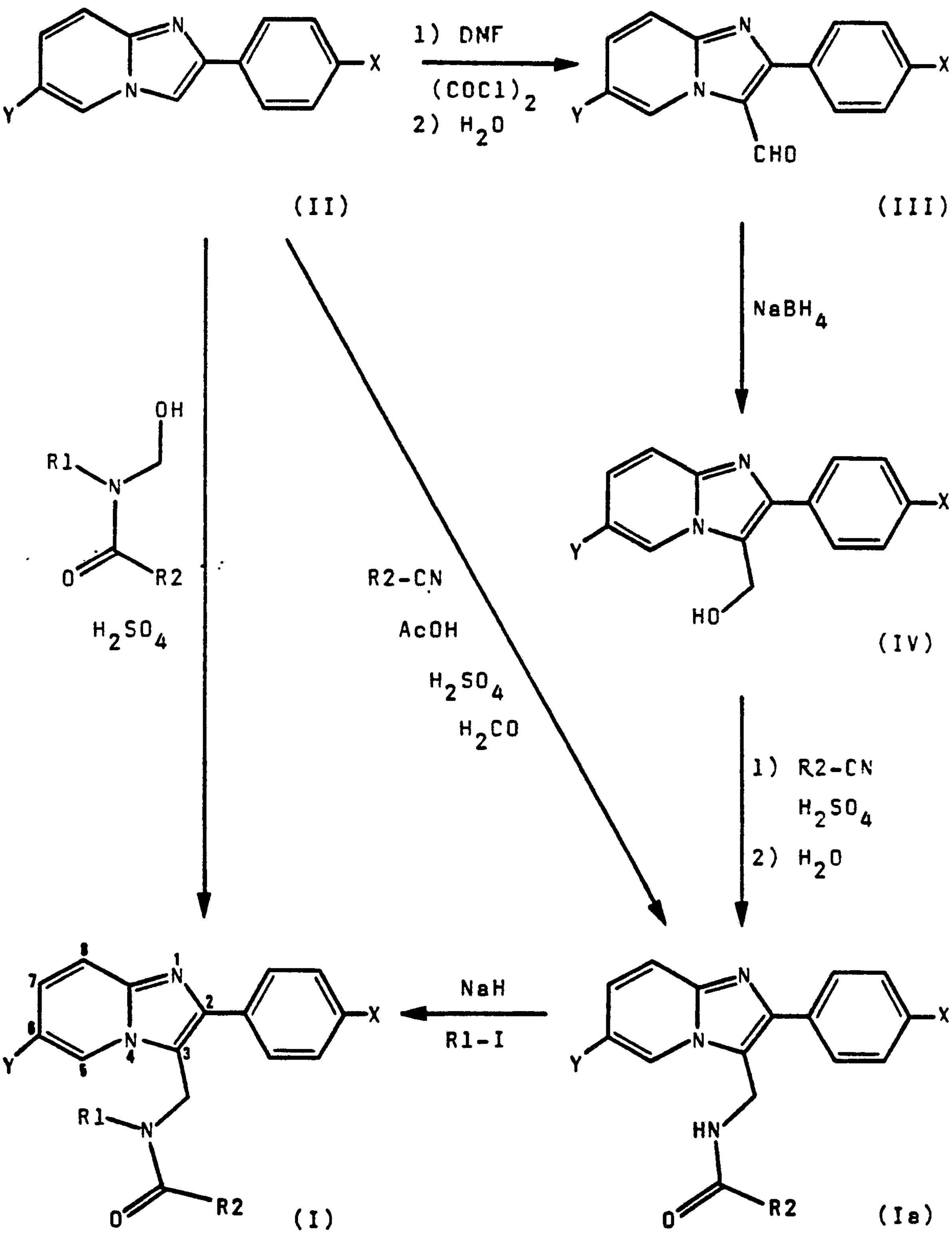


Le composé de départ est une phényl-2 imidazo[1,2-a]pyridine de formule II portant les substituants X et Y tels que définis ci-dessus.

On peut effectuer d'abord une formylation de ce composé (II), par exemple au moyen du réactif que l'on obtient par action du chlorure d'oxalyle sur le diméthylformamide. On obtient après hydrolyse un aldéhyde de formule III que l'on réduit en l'alcool correspondant, de formule IV, de manière connue, par exemple par action d'un borohydrure de métal alcalin.

Cet alcool (IV) est ensuite mis à réagir avec un nitrile de formule R2-CN en milieu sulfurique. On obtient ainsi après hydrolyse un composé de formule Ia dont le substituant $R^1$ est nécessairement l'hydrogène. Si on le désire, on peut alors alkyler ou benzyler ce composé de manière connue, par exemple avec un iodure de

formule R1-I en présence d'hydrure de sodium, pour aboutir à un composé de formule I dans laquelle R1 est un groupe alkyle ou benzyle.

On peut également préparer les composés de formule I en une seule étape, en faisant réagir le composé (II) de départ avec un amide de formule

```
R2-C-N-R1
   ‖ |
   O CH2OH
```

dans laquelle R1 et R2 sont tels que définis ci-dessus. La réaction s'effectue à température ambiante, en présence d'acide sulfurique concentré et éventuellement avec un cosolvant tel que l'acide acétique glacial.

Enfin on peut encore préparer des composés de formule Ia en faisant réagir le composé (II) de départ avec un nitrile de formule R2-CN et le paraformaldéhyde, à chaud, en milieu acétique et en présence d'acide sulfurique.

Le composé (I) obtenu après hydrolyse peut, si on le désire, être ensuite alkylé ou benzylé comme indiqué plus haut.

Les exemples suivants illustrent la préparation de quelques composés selon l'invention. La microanalyse et les spectres IR et RMN confirment les structures des produits obtenus.

**Exemple 1** (Chloro-4 phényl)-2 pentanoylaminométhyl-3 méthyl-6 imidazo[1,2-a]pyridine.

a) Dans un ballon maintenu entre -30 et -40°C et contenant 150 ml de diméthylformamide on introduit au goutte à goutte 63 g, soit 43 ml (0,5 mole) de chlorure d'oxalyle, tout en agitant. On laisse revenir à température ambiante puis on ajoute une suspension de 40 g (0,165 mole) de (chloro-4 phényl)-2 méthyl-6 imidazo[1,2-a]pyridine dans 150 ml de diméthylformamide et on poursuit l'agitation durant 24 heures. On sépare le précipité par filtration, on le met en suspension dans 1 l d'eau et, tout en agitant cette suspension, on ajoute 350 ml d'ammoniaque.

On extrait le mélange avec du chlorure de méthylène, on sépare la phase organique, on la lave à l'eau, on la sèche sur sulfate de magnésium et on l'évapore. On rince le résidu avec de l'éther et on le recristallise dans le méthanol. On obtient un solide blanc. F = 175 - 176°C.

b) On prépare une suspension de 40 g (0,148 mole) de l'aldéhyde précédemment obtenu dans 500 ml de méthanol et on ajoute rapidement une solution de 3,5 g de borohydrure de sodium dans 15 ml d'eau. On note un échauffement du mélange et un fort dégagement gazeux.

On agite le mélange pendant 24 heures puis on l'évapore à sec, on reprend le résidu par de l'eau, on ajuste le pH à 8 avec de l'acide chlorhydrique dilué, on filtre le précipité, on le lave et on le sèche. F = 214 - 215°C.

c) On introduit dans un ballon 6,12 ml (0,0588 mole) de valéronitrile et 32 ml d'acide sulfurique. On agite le mélange et lui ajoute par petites portions 8 g (0,0293 mole) de l'alcool précédemment préparé, et on continue d'agiter jusqu'à dissolution complète.

On ajoute alors de l'eau glacée, puis de l'ammoniaque jusqu'à pH>7 et on extrait avec du chlorure de méthylène. On sépare la phase organique, on la lave, on la sèche et on l'évapore. On lave le résidu à l'éther, puis on le purifie par chromatographie sur silice en éluant avec un mélange 96/4 de chlorure de méthylène/méthanol. Le produit final fond à 172 - 173°C.

**Exemple 2** (Chloro-4 phényl)-2 N-méthyl-pentanoylamino-méthyl-3 méthyl-6 imidazo[1,2-a]pyridine.

Dans un ballon de 150 ml on introduit 1,27 g (0,0264 mole) d'hydrure de sodium à 50 % dans de l'huile, on le lave deux fois avec du pentane, et on ajoute 30 ml de tétrahydrofuranne et 1,5 ml de diméthylformamide.

Puis, sous argon, on introduit 4,7 g (0,0132 mole) de l'amide obtenu selon l'exemple 1, en solution dans 150 ml de tétrahydrofuranne, puis 1,64 ml (0,0264 mole) de iodométhane.

On note un dégagement gazeux. On suit l'évolution de la réaction par chromatographie sur couche mince de silice. Lorsqu'il n'y a plus de produit de départ, on ajoute du méthanol pour détruire l'excès d'hydrure de sodium.

On évapore le mélange à sec, on le reprend par de l'eau et du chlorure de méthylène, on sépare la phase organique, on la lave à l'eau, on la sèche et on l'évapore.

On reprend le résidu avec de l'éther, puis on le purifie par chromatographie sur colonne de silice en éluant avec un mélange 97/3 de chlorure de méthylène/méthanol. Le produit final fond à 158 - 160°C.

**Exemple 3** (chloro-4 phényl)-2 (méthyl-2 propanoylamino-méthyl)-3 méthyl-6 imidazo[1,2-a]pyridine.

Dans un ballon de 150 ml on introduit 1,5 g (0,05 mole) de paraformaldéhyde, 50 ml d'acide acétique, 4,55 ml (0,05 mole) d'isobutyronitrile et 2 ml d'acide sulfurique concentré, et on chauffe le tout à 70°C jusqu'à dissolution. On ajoute alors 4 g (0,0165 mole) de (chloro-4 phényl)-2 méthyl-6 imidazo[1,2-a]pyridine et 15 ml d'acide acétique et on chauffe au bain-marie en surveillant l'évolution de la réaction par chromatographie sur couche mince.

Lorsque le produit de départ a presque complètement disparu on laisse refroidir, on filtre le précipité qui s'est formé, on le reprend avec de l'eau et de l'ammoniaque et on extrait avec du chlorure de méthylène.

On lave la phase organique à l'eau, on la sèche et on l'évapore. On purifie le résidu sur colonne de silice en éluant avec un mélange 95/5 de chlorure de méthylène/méthanol. Le composé obtenu fond à 211 - 212° C.

**Exemple 4** (Chloro-4 phényl)-2 benzoylaminométhyl-3 méthyl-6 imidazo[1,2-a]pyridine.

A une solution de 4,54 g (0,03 mole) de N-hydroxyméthyl-benzamide dans 50 ml d'acide acétique glacial on ajoute 2 g d'acide sulfurique concentré puis, après 15 minutes de chauffage à 50° C, on ajoute 4,8 g (0,02 mole) de (chloro-4 phényl)-2 méthyl-6 imidazo[1,2-a]pyridine.

On chauffe le mélange au reflux pendant 6 heures, puis on l'évapore à sec. On reprend le résidu avec de l'eau, on ajoute de l'ammoniaque jusqu'à obtenir un pH basique. On filtre le précipité, on le lave à l'eau, on le sèche et on le recristallise dans 400 ml de méthanol. On obtient des cristaux blancs qui fondent à 247 - 248° C.

**Exemple 5** (Chloro-4 phényl)-2 (oxo-2 pyrrolidinyl-l)méthyl-3 méthyl-6 imidszo[1,2-a]pyridine.

On prépare un mélange intime de 4,8 g (0,02 mole) de (chloro-4 phényl)-2 méthyl-6 imidazo[1,2-a]pyridine et de 2,53 g (0,022 mole) de N-hydroxyméthyl-pyrrolidinone-2, puis on l'introduit dans 20 ml d'acide sulfurique concentré. On agite la solution une journée à température ambiante, puis on la verse dans 800 ml d'eau, et on ajoute 55 ml d'ammoniaque. On extrait le lactame au moyen de chlorure de méthylène, on lave et sèche la phase organique, on l'évapore et on purifie le résidu par chromatographie en éluant avec un mélange 70/30 d'acétate d'éthyle/toluène. Le solide blanc obtenu fond à 186 - 187,5° C.

Le tableau ci-après décrit les structures et les points de fusion d'autres composés selon l'invention.

**Tableau**

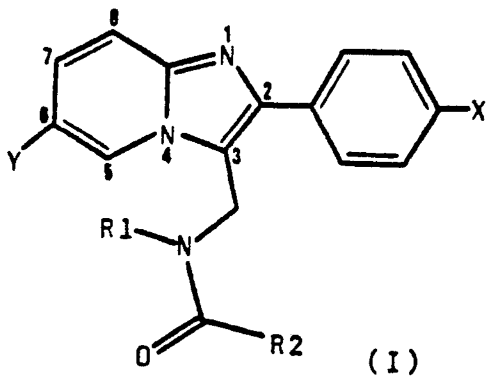

(I)

| Composé | Y | X | R1 | R2 | F (° C) |
|---|---|---|---|---|---|
| 1 | H | Cl | H | $CH_3$ | 205-206 |
| 2 | H | $CH_3$ | H | $CH_3$ | 174-175 |
| 3 | $CH_3$ | $CH_3$ | H | $CH_3$ | 218-218,5 |
| 4 | $CH_3$ | Cl | H | $CH_3$ | 230-231 |
| 5 | $CH_3$ | Cl | H | $C_2H_5$ | 190-191 |
| 6 (Ex. 3) | $CH_3$ | Cl | H | $i\text{-}C_3H_7$ | 211-212 |
| 7 | $CH_3$ | Cl | H | $n\text{-}C_3H_7$ | 185-187 |
| 8 (Ex. 1) | $CH_3$ | Cl | H | $n\text{-}C_4H_9$ | 172-173 |
| 9 | $CH_3$ | Cl | H | $i\text{-}C_4H_9$ | 192-194 |
| 10 | $CH_3$ | Cl | H | $t\text{-}C_4H_9$ | 226-228 |
| 11 | $CH_5$ | Cl | H | $C_6H_5$ | 247-248 |
| 12 | $CH_3$ | H | H | $C_6H_5$ | 209-211 |
| 13 | $CH_3$ | Cl | H | 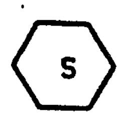 | 209-210 |
| 14 | $CH_3$ | $CH_3$ | $CH_3$ | $C_2H_5$ | 151-152 |
| 15 | $CH_3$ | Cl | $CH_3$ | $CH_3$ | 199-200 |
| 16 | $CH_3$ | Cl | $C_2H_5$ | $CH_3$ | 293-294 |
| 17 | $CH_3$ | Cl | $n\text{-}C_4H_9$ | $CH_3$ | 182-183 |
| 18 | $CH_3$ | Cl | $CH_2C_6H_5$ | $CH_3$ | 149-150 |

| | | | | | |
|---|---|---|---|---|---|
| 19 | $CH_3$ | Cl | $CH_3$ | $C_2H_5$ | 210-212 |
| 20 | $CH_3$ | Cl | $CH_3$ | $n\text{-}C_3H_7$ | 193-195 |
| 21 | $CH_3$ | Cl | $CH_3$ | $i\text{-}C_3H_7$ | 152-154 |
| 22 (Ex. 2) | $CH_3$ | Cl | $CH_3$ | $n\text{-}C_4H_9$ | 158-160 |
| 23 | $CH_3$ | Cl | $CH_3$ | $t\text{-}C_4H_9$ | 160-162 |
| 24 | $CH_3$ | Cl | $CH_3$ | $C_6H_5$ | 198-199 |
| 25 (Ex. 5) | $CH_3$ | Cl | $-(CH_2)_3-$ | | 186-187,5 |
| 26 | $CH_3$ | Cl | $-(CH_2)_4-$ | | 145-146 |
| 27 | $CH_3$ | Cl | $-(CH_2)_5-$ | | 150-151 |
| 28 | $CH_3$ | Cl | $CH_3$ | $i\text{-}C_4H_9$ | 185-186 |
| 29 | $CH_3$ | Cl | $CH_3$ | $n\text{-}C_5H_{11}$ | 127-129 |
| 30 | $CH_3$ | Cl | $CH_3$ | 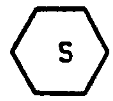 | 178-180 |
| 31 | $CH_3$ | Cl | $CH_3$ | $n\text{-}C_6H_{13}$ | 104-105 |
| 32 | $CH_3$ | Cl | $CH_3$ | $CH_2C_6H_5$ | 126-127 |
| 33 | $CH_3$ | Cl | $CH_3$ | $CH=CHCH_3$ | 225-227 |
| 34 | $CH_3$ | $OCH_3$ | $CH_3$ | $n\text{-}C_3H_7$ | 135-136 |
| 35 | $CH_3$ | $OCH_3$ | $CH_3$ | $i\text{-}C_4H_9$ | 93-95 |
| 36 | $CH_3$ | $NO_2$ | $CH_3$ | $n\text{-}C_3H_7$ | 154-156 |
| 37 | $CH_3$ | $NO_2$ | $CH_3$ | $i\text{-}C_4H_9$ | 157-159 |
| 38 | $CH_3$ | $SO_2CH_5$ | $CH_3$ | $n\text{-}C_3H_7$ | 182-183 |
| 39 | $CH_3$ | $SO_2CH_3$ | $CH_3$ | $i\text{-}C_4H_9$ | 120-122 |
| 40 | $CH_3$ | H | $CH_3$ | $n\text{-}C_3H_7$ | 125-126,5 |
| 41 | $CH_3$ | H | $CH_3$ | $i\text{-}C_4H_9$ | 154-156 |
| 42 | $CH_3$ | $NHCOCH_3$ | $CH_3$ | $n\text{-}C_3H_7$ | 110-111 |
| 43 | $CH_3$ | $NHCOCH_3$ | $CH_3$ | $i\text{-}C_4H_9$ | 183-185 |
| 44 | $CH_3$ | $N(COCH_3)_2$ | $CH_3$ | $n\text{-}C_3H_7$ | 117-118 |
| 45 | $CH_3$ | $NH_2$ | $CH_3$ | $i\text{-}C_4H_9$ | 177,5-178,5 |
| 46 | $CH_3$ | $NHCH_3$ | $CH_3$ | $i\text{-}C_4H_9$ | 176-177 |
| 47 | $CH_3$ | $N(CH_3)_2$ | $CH_3$ | $i\text{-}C_4H_9$ | 117-118 |
| 48 | $CH_3$ | Cl | $CH_3$ | $4\text{-}Cl\text{-}C_6H_4$ | 175-177 |
| 49 | $CH_3$ | $n\text{-}C_4H_9$ | $CH_3$ | $i\text{-}C_4H_9$ | 72,5-73 |
| 50 | $CH_3$ | $C_2H_5$ | $CH_3$ | $i\text{-}C_4H_9$ | 99-101 |
| 51 | $CH_3$ | $C_2H_5$ | $CH_3$ | $n\text{-}C_3H_7$ | 108-110 |
| 52 | $CH_3$ | F | $CH_3$ | $i\text{-}C_4H_9$ | 184-185 |
| 53 | $CH_3$ | F | $CH_3$ | $n\text{-}C_3H_7$ | 157-158 |
| 54 | $CH_3$ | $SCH_3$ | $CH_3$ | $i\text{-}C_4H_9$ | 102-104 |
| 55 | $CH_3$ | $SCH_3$ | $CH_3$ | $n\text{-}C_3H_7$ | 128-129 |
| 56 | $CH_3$ | $CH_3$ | $CH_3$ | $i\text{-}C_4H_9$ | 129-131 |
| 57 | $CH_3$ | $CH_3$ | $CH_3$ | $n\text{-}C_3H_7$ | 145-147 |
| 58 | H | Cl | $CH_3$ | $i\text{-}C_4H_9$ | 90-91 |
| 59 | H | Cl | $CH_3$ | $n\text{-}C_3H_7$ | 171-173 |
| 60 | Cl | Cl | $CH_3$ | $i\text{-}C_4H_9$ | 176-177 |
| 61 | Cl | Cl | $CH_3$ | $n\text{-}C_3H_7$ | 188-189 |
| 62 | Cl | F | $CH_3$ | $n\text{-}C_3H_7$ | 154-155 |
| 63 | Cl | F | $CH_3$ | $i\text{-}C_4H_9$ | 173-174 |
| 64 | Cl | $C_2H_5$ | $CH_3$ | $n\text{-}C_3H_7$ | 134-136 |
| 65 | Cl | $C_2H_5$ | $CH_3$ | $i\text{-}C_4H_9$ | 111-112 |
| 66 | Cl | $CH_3$ | $CH_3$ | $n\text{-}C_3H_7$ | 164-165 |
| 67 | Cl | $CH_3$ | $CH_3$ | $i\text{-}C_4H_9$ | 154-155 |
| 68 | Cl | $OCH_3$ | $CH_3$ | $n\text{-}C_3H_7$ | 152-153 |
| 69 | Cl | $OCH_3$ | $CH_3$ | $i\text{-}C_4H_9$ | 131-132 |
| 70 | Cl | $SCH_3$ | $CH_3$ | $n\text{-}C_3H_7$ | 139-141 |
| 71 | Cl | $SCH_3$ | $CH_3$ | $i\text{-}C_4H_9$ | 135-136 |
| 72 | $CH_3$ | Cl | H | $n\text{-}C_5H_{11}$ | 164-166 |
| 73 | $CH_3$ | Cl | H | $n\text{-}C_6H_{13}$ | 159-161 |
| 74 | $CH_3$ | Cl | H | $CH_2C_6H_5$ | 194-195 |
| 75 | $CH_3$ | Cl | H | $CH_2CH=CH_2$ | 179-180 |
| 76 | $CH_3$ | Cl | H | $4\text{-}Cl\text{-}C_6H_4$ | 252-254 |
| 77 | $CH_3$ | Cl | H | $CCl_3$ | 228-230 |
| 78 | $CH_3$ | $n\text{-}C_4H_9$ | H | $i\text{-}C_4H_9$ | 118,5-119,5 |
| 79 | $CH_3$ | F | H | $i\text{-}C_4H_9$ | 188-190 |

| | | | | | |
|---|---|---|---|---|---|
| 80 | $CH_3$ | F | H | n-$C_3H_7$ | 140-141 |
| 81 | $CH_3$ | $SCH_3$ | H | i-$C_4H_9$ | 183-184 |
| 82 | $CH_3$ | $SCH_3$ | H | n-$C_3H_7$ | 170-172 |
| 83 | $CH_3$ | $CH_3$ | H | i-$C_4H_9$ | 161-162 |
| 84 | $CH_3$ | $CH_3$ | H | n-$C_3H_7$ | 170-171 |
| 85 | H | Cl | H | i-$C_4H_9$ | 175-176 |
| 86 | H | Cl | H | n-$C_3H_7$ | 198-199 |
| 87 | Cl | Cl | H | i-$C_4H_9$ | 223,5-224 |
| 88 | Cl | Cl | H | n-$C_3H_7$ | 217-218 |
| 89 | $CH_3$ | $C_2H_5$ | H | n-$C_3H_7$ | 150-151 |
| 90 | $CH_3$ | $C_2H_5$ | H | i-$C_4H_9$ | 154-155 |
| 91 | $CH_3$ | $OCH_3$ | H | n-$C_3H_7$ | 143-144 |
| 92 | $CH_3$ | $OCH_3$ | H | i-$C_4H_9$ | 171-172 |
| 93 | $CH_3$ | $SO_2CH_3$ | H | n-$C_3H_7$ | 209-211 |
| 94 | $CH_3$ | $SO_2CH_3$ | H | i-$C_4H_9$ | 175-176 |
| 95 | $CH_3$ | $NO_2$ | H | n-$C_3H_7$ | 210-212 |
| 96 | $CH_3$ | $NO_2$ | H | i-$C_4H_9$ | 220-222 |
| 97 | $CH_3$ | H | H | n-$C_3H_7$ | 155-156 |
| 98 | $CH_3$ | H | H | i-$C_4H_9$ | α) 137-139<br>β) 168-171 |
| 99 | $CH_3$ | $NHCOCH_3$ | H | n-$C_3H_7$ | 204-205 |
| 100 | $CH_3$ | $NHCOCH_3$ | H | i-$C_4H_9$ | 183-185 |
| 101 | Cl | F | H | n-$C_3H_7$ | 193-194 |
| 102 | Cl | F | H | i-$C_4H_9$ | 208-209 |
| 103 | Cl | $C_2H_5$ | H | n-$C_3H_7$ | 181-182 |
| 104 | Cl | $C_2H_5$ | H | i-$C_4H_9$ | 188-189 |
| 105 | Cl | $CH_3$ | H | n-$C_3H_7$ | 191-192 |
| 106 | Cl | $CH_3$ | H | i-$C_4H_9$ | 203-204 |
| 107 | Cl | $OCH_3$ | H | n-$C_3H_7$ | 183-184 |
| 108 | Cl | $OCH_3$ | H | i-$C_4H_9$ | 195-196 |
| 109 | Cl | $SCH_3$ | H | n-$C_3H_7$ | 204-205 |
| 110 | Cl | $SCH_3$ | H | i-$C_4H_9$ | 207-208 |
| 111 | $CH_3$ | $SCH_3$ | n-$C_3H_7$ | i-$C_4H_9$ | 90-91(*) |
| 112 | $CH_3$ | $SC_2H_5$ | H | i-$C_4H_9$ | 149-150 |
| 113 | $CH_3$ | $SC_2H_5$ | $CH_3$ | i-$C_4H_9$ | 118,5-119,5 |

Notes:

n-$C_3H_7$, n-$C_4H_9$, n-$C_zH_{2z+1}$, etc, désignent des chaînes linéaires en $C_3$, $C_4$, $C_z$, etc.

i-$C_3H_7$ et i-$C_4H_9$ désignent les chaînes isopropyle et isobutyle, respectivement.

t-$C_4H_9$ désigne la chaîne tertiobutyle.

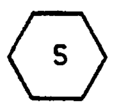 désigne le groupe cyclohexyle.

$C_6H_5$ et 4-Cl-$C_6H_4$ désignent les groupes phényle et parachlorophényle, respectivement.

α) et β): les deux points de fusion du composé n° 98 correspondent à deux modifications cristallines.

(*): le composé n°111 est sous forme de chlorhydrate.

Les composés de l'invention ont fait l'objet d'essais pharmacologiques qui ont mis en évidence leur intérêt comme substances à activités thérapeutiques.

Toxicité aiguë.

Elle a été déterminée chez la souris par voie intrapéritonéale. Les DL 50 sont supérieures à 500 mg/kg.

Antagonisme vis-à-vis des convulsions cloniques induites par le Cardiezol® chez la souris.

L'essai est inspiré du protocole décrit par Goodman et al., J. Pharm. Exp. Ther., 108, 168-176. Les souris reçoivent les produits à tester, ou le solvant seul, par voie intrapéritonéale 30 minutes avant l'injection de 35 mg/kg de Cardiazol® par voie intraveineuse. Les animaux sont ensuite observés pendant une heure et, pour chaque lot, le pourcentage de souris présentant des convulsions cloniques est noté (100 % de convulsions cloniques et 10 a 20 % de convulsions toniques chez les animaux de contrôle).

Pour chaque dose, on calcule le pourcentage de protection par rapport aux animaux de contrôle, ce qui permet de déterminer graphiquement la $DA_{50}$, dose qui protège 50 % des animaux vis-à-vis des effets convulsivants du Cardiazol®. Les $DA_{50}$ des composés de l'invention se situent entre 0,01 et 30 mg/kg.

Test "d'enfouissement" chez la souris ("Burying test").

Ce test est inspiré de la méthode décrite par Pinel J.P.J., Treit D., Ladak F. et MacLennan A.J. dans Animal learning and behavior, 8,447-451, (1980).

La présence de corps étrangers dans l'environnement habituel d'un animal constitue une situation aversive à

laquelle l'animal réagit en enfouissant l'objet de l'agression (billes de verre) dans la sciure de sa cage.

Les anxiolytiques ont pour effet de diminuer l'appréhension causée par la présence étrangère: les animaux enfouissent moins. On compte alors le nombre de billes restées non enfouies.

Les produits à étudier sont administrés à des souris mâles de souche CDI (Charles River) 30 minutes (voie intrapéritonéale) ou 60 minutes (voie orale) avant que ces dernières soient placées dans des cages contenant 25 billes de verre. Au bout de 30 minutes on compte le nombre de billes restées non enfouies. Un pourcentage est calculé entre les animaux traités et les animaux témoins.

On détermine ainsi la $DA_{50}$, dose active 50 %, qui est la dose de composé (en mg/kg) diminuant de moitié le nombre de billes enfouies, en comparaison avec les animaux témoins. Les $DA_{50}$ des composés de l'invention se situent entre 0,1 et 30 mg/kg par voie intrepéritonéale.

Action sur l'électrocorticogramme du rat curarisé ventilé.

L'activité sédative ou hypnotique des composés a été déterminée par l'observation de leur action sur l'électrocorticogramme du rat selon la méthode décrite par H. Depoortere, Rev. E.E.G. Neurophysiol.,10, 3, 207-214 (1980) et par H. Depoortere et M. Decobert, J. Pharmscol. (Paris), 14, 2, 195-265 (1983).

Les produits à étudier ont été administrés par voie intrapéritonéale aux doses croissantes de 1 à 30 mg/kg. Ils induisent des tracés de sommeil à partir de doses allant de 0,01 à 30 mg/kg.

Effets sur la durée du "sommeil" induit par le hydroxy-4 butyrate de sodium.

Cette action a été déterminée par l'influence d'un composé sur la durée du "sommeil" induit par le hydroxy-4 butyrate de sodium (GHB) chez le rat curarisé.

Les animaux utilisés sont des rats mâles de souche Charles River de 200 ± 20 g. Les animaux, curarisés par l'alloférine à raison de 1 mg/kg par voie i.p., sont placés sous respiration artificielle à l'aide d'un masque appliqué sur le museau (fréquence respiratoire = 50/minute; volume respiratoire = 14 ml).

L'oesophage est préalablement ligaturé afin d'éviter l'entrée de l'air dans l'estomac.

Des électrodes corticales front-pariétales et occipitales permettent l'enregistrement de l'activité électrocorticographique sur un polygraphe Grass modèle 79 P à la vitesse de 6 mm/sec.

La préparation de l'animal est effectuée sous anesthésie locale (xylocaine à 2 %). Les rats sont maintenus tout au long de l'expérience à température constante (37,5° C). Dix minutes après la fin de la préparation du rat, une dose de 200 mg/kg de hydroxy-4 butyrate de sodium est injectée par voie intraveineuse au niveau de la queue.

Une dose de 10 mg/kg du composé à étudier est administrée par voie intrepéritonéale 3 minutes après l'administration du hydroxy-4 butyrate de sodium.

L'évaluation des tracés s'effectue par périodes de 15 minutes durant 75 minutes après l'injection de GHB. Durant cette période d'analyse, la durée totale du "sommeil" est éterminée. Une série de 15 témoins permet de préciser la durée du "sommeil GHB".

L'analyse statistique des résultats est réalisée à l'aide du test "U" de Mann-Whitney.

Certains composés réduisent les effets du GHB (jusqu'à 40 % de diminution de la durée du sommeil à la dose de 10 mg/kg), tandis que d'autres potentialisent ces effets (jusqu'à 40 % d'augmentation à la dose de 10 mg/kg). On constate également que les effets peuvent être opposés selon que les composés sont administrés à fortes doses ou à faibles doses.

Les résultats de ces différents test montrent que les composés de l'invention possèdent des propriétés anxiolytiques, inductrices de sommeil, hypnotiques et anticonvulsivantes; les composés de l'invention sont utiles pour le traitement les états d'anxiété, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens et pour le traitement des encéphalopathies métaboliques.

Les composés de l'invention peuvent être présentés sous toute forme appropriée pour l'administration par voie orale, ou parentérale, par exemple sous forme de comprimés, de dragées, de gélules, de solutions buvables ou injectables etc, en association avec tout excipient approprié.

La posologie quotidienne peut aller de 0,1 à 100 mg.

**Revendications** pour les états contractants: BE, CH, DE, FR, GB, IT, LI, LU, NL, SE

1. Composés répondant à la formule générale I

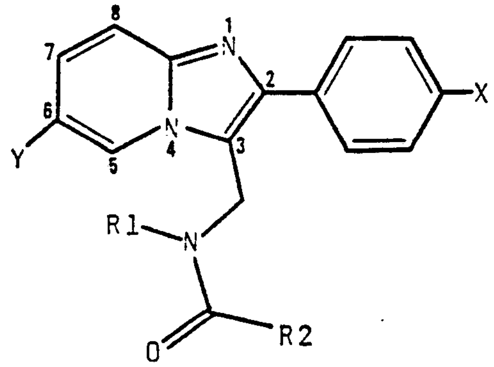

(I)

dans laquelle

X représente l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino,

Y représente l'hydrogène, le chlore ou un groupe méthyle,

R1 représente l'hydrogène ou un groupe alkyle en $C_1$-$C_4$ linéaire ou ramifié, ou un groupe benzyle,

R2 représente un groupe alkyle en $C_1$-$C_6$ linéaire ou ramifié, un groupe cyclohexyle, trichlorométhyle, propène-1 yle, allyle, phényle, chloro-4 phényle ou benzyle, ou bien encore

R1 et R2 représentent ensemble une chaîne aliphatique en $C_3$-$C_5$,

ainsi que leurs sels d'addition à des acides acceptables en pharmacologie.

2. Composés selon la revendication 1, caractérisés en ce que X représente le chlore ou un groupe méthyle ou méthylthio, Y représente le chlore ou un groupe méthyle, R1 représente l'hydrogène ou un groupe méthyle, et R2 représente un groupe propyle ou isobutyle.

3. Procédé de préparation des composés de formule I tels que spécifiés dans la revendication 1, caractérisé en ce qu'on effectue une formylation d'un composé de formule II

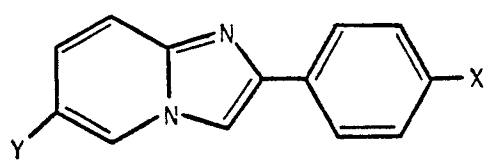

(II)

dans laquelle X et Y sont tels que définis dans la revendication 1,

au moyen du réactif obtenu par action du chlorure d'oxalyle sur le diméthylformamide pour obtenir après hydrolyse, un aldéhyde de formule III

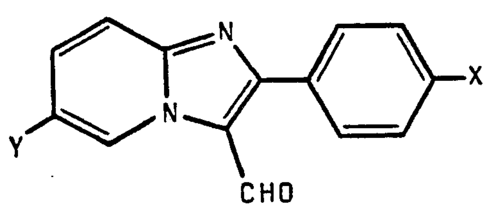

(III)

que l'on réduit de manière connue, de préférence par action d'un borohydrure de métal alcalin, en un alcool de formule IV

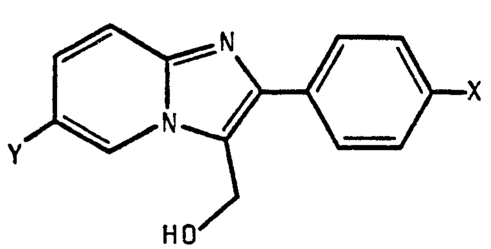

(IV)

puis on traite ce dernier en milieu sulfurique avec un nitrile de formule R2-CN, dans laquelle R2 est tel que défini dans la revendication 1, pour obtenir, après hydrolyse, un amide de formule I dans laquelle R1 représente l'hydrogène et, si on le souhaite, on alkyle ce dernier de manière connue avec un composé de formule R1-I, dans laquelle R1 est tel que défini dans la revendication 1, en présence d'hydrure de sodium.

4. Procédé de préparation des composés de formule I tels que spécifiés dans la revendication 1, caractérisé en ce qu'on fait réagir un composé de formule II

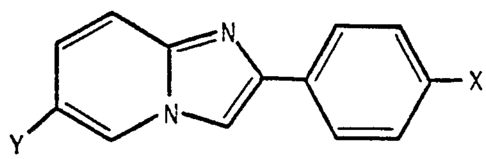

(II)

avec un amide de formule

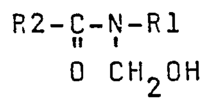

dans laquelle R1 et R2 sont tels que définis dans la revendication 1, à température ambiante, en présence d'acide sulfurique et éventuellement avec un cosolvant tel que l'acide acétique glacial.

5. Procédé de préparation des composés de formule I tels que spécifiés dans la revendication 1, caractérisé en ce qu'on fait réagir un composé de formule II

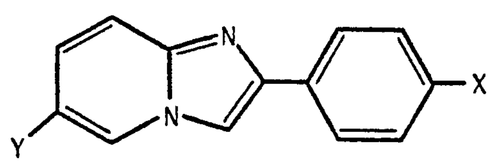

(II)

avec le paraformaldéhyde et avec un nitrile de formule R2-CN, dans laquelle R2 est tel que défini dans la revendication 1, à chaud, en milieu acétique et en présence d'acide sulfurique et, après hydrolyse, si on le souhaite, on alkyle le composé obtenu, de manière connue avec un composé de formule R1-I, dans laquelle R1 est tel que défini dans la revendication 1, en présence d'hydrure de sodium.

6. Composition pharmaceutique, caractérisée en ce qu'elle contient un composé selon la revendication 1 ou 2, en association avec un excipient approprié.

**Revendications** pour l'état contractant : AT

1. Procédé de préparation de composés répondant à la formule générale I

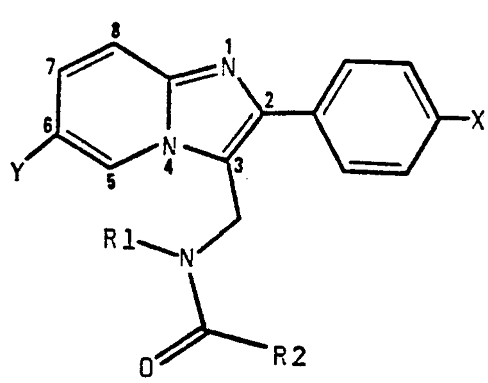

(I)

dans laquelle

X représente l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino,

Y représente l'hydrogène, le chlore ou un groupe méthyle,

R1 représente l'hydrogène ou un groupe alkyle en $C_1$-$C_4$ linéaire ou ramifié, ou un groupe benzyle,

R2 représente un groupe alkyle en $C_1$-$C_6$ linéaire ou ramifié, un groupe cyclohexyle, trichlorométhyle, propène-1 yle, allyle, phényle, chloro-4 phényle ou benzyle, ou bien encore

R1 et R2 représentent ensemble une chaîne aliphatique en $C_3$-$C_5$,

procédé caractérisé en ce qu'on effectue une formylation d'un composé de formule

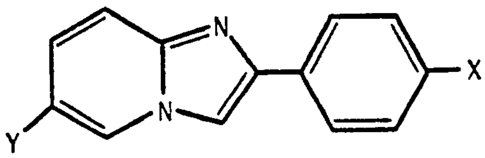

(II)

dans laquelle X et Y sont tels que définis ci-dessus, au moyen du réactif obtenu par action du chlorure d'oxalyle sur le diméthylformamide, pour obtenir, après hydrolyse, un aldéhyde de formule III

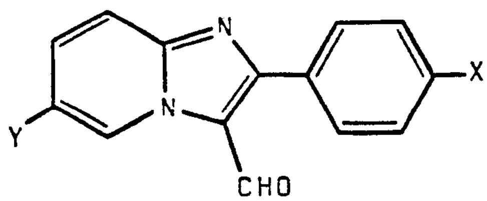

(III)

que l'on réduit de manière connue, de préférnce par action d'un borohydrure de métal alcalin en un alcool de formule IV

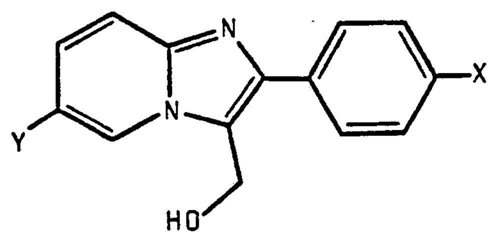

(IV)

puis on traite ce dernier en milieu sulfurique avec un nitrile de formule R2-CN, dans laquelle R2 est tel que défini ci-dessus, pour obtenir, après hydrolyse, un amide de formule I dans laquelle R1 représente l'hydrogène et, si on le souhaite, on alkyle ce dernier de manière connue avec un composé de formule R1-I, dans laquelle R1 est tel que défini ci-dessus, en présence d'hydrure de sodium.

2. Procédé de préparation des composés de formule I tels que spécifiés dans la revendication 1, caractérisé en ce qu'on fait réagir un composé de formule II

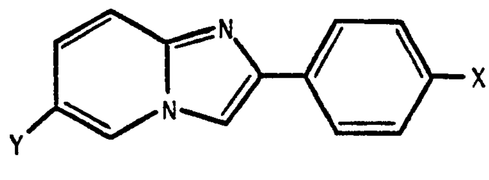

(II)

avec un amide de formule

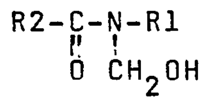

dans laquelle R1 et R2 sont tels que définis dans la revendication 1, à température ambiante, en présence d'acide sulfurique et éventuellement avec un cosolvant tel que l'acide acétique glacial.

3. Procédé de préparation des composés de formule I tels que spécifiés dans la revendication 1, caractérisé en ce qu'on fait réagir un composé de formule II

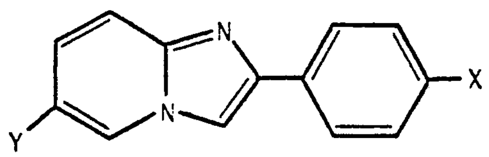

(II)

avec le paraformaldéhyde et avec un nitrile de formule R2-CN, dans laquelle R2 est tel que défini dans la revendication 1, à chaud, en milieu acétique et en présence d'acide sulfurique et, après hydrolyse, si on le souhaite, on alkyle le composé obtenu, de manière connue avec un composé de formule R1-I, dans laquelle R1 est tel que défini dans la revendication 1, en présence d'hydrure de sodium.

**Patentansprüche** für die Vertragsstaaten BE, CH, DE, FR, GB, IT, LI, LU, NL, SE

1. Verbindungen der allgemeinen Formel I

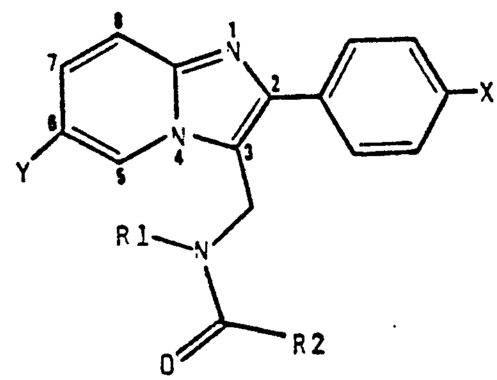

(I)

in welcher X für Wasserstoff, ein Halogen oder eine $C_1$-$C_4$-Alkyl-, eine Methoxy-, Methylthio-, Ethylthio-, Methylsulfonyl-, Nitro-, Amino-, Methylamino-, Dimethylamino-, Acetylamino- oder Diacetylaminogruppe,

Y für Wasserstoff, Chlor oder eine Methylgruppe,

R1 für Wasserstoff oder eine geradkettige oder verzweigte $C_1$-$C_4$-Alkylgruppe oder eine Benzylgruppe und

R2 für eine lineare oder verzweigte $C_1$-$C_6$-Alkylgruppe, eine Cyclohexyl-, Trichlormethyl-, Propen-1-yl-, Allyl-, Phenyl-, 4-Chlor-phenyl oder eine Benzylgruppe steht oder aber

R1 und R2 gemeinsam eine aliphatische $C_3$-$C_5$-Kette bedeuten,

sowie ihre Säureadditionssalze mit pharmakologisch verwendbaren säuren.

2. Verbindungen nach Anspruch 1, dadurch gekennzeichnet, daß X für Chlor oder eine Methyl- oder Methylthiogruppe, Y für Chlor oder eine Methylgruppe, R1 für Wasserstoff oder eine Methylgruppe und R2 für eine propyl- oder Isobutylgruppe stehen.

3. Verfahren zur Herstellung von Verbindungen der Formel I wie sie in Anspruch 1 angegeben sind, dadurch gekennzeichnet, daß man eine Formylierung einer Verbindung der Formel II

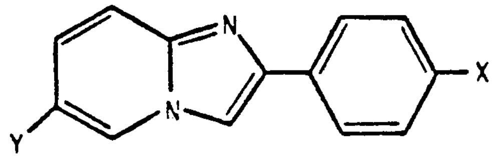

(II)

in welcher X und Y die in Anspruch 1 angegebene Bedeutung haben, mit Hilfe eines durch Einwirkung von Oxalylchlorid auf Dimethylformamid erhaltenen Reagens durchführt, um, nach einer Hydrolyse, einen Aldehyd der Formel III

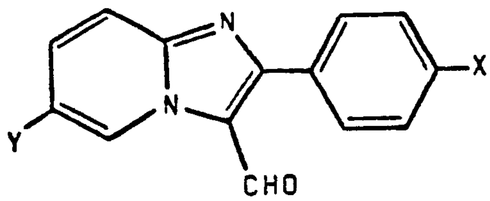

(III)

zu gewinnen, den man in bekannter Weise, vorzugsweise durch Einwirkung eines Alkalimetallborhydrids, reduziert zu einem Alkohol der Formel IV

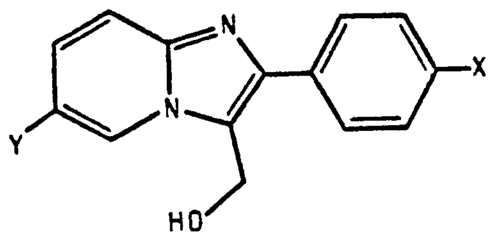

(IV)

worauf man diesen letzeren in schwefelsaurem Medium mit einem Nitril der Formel R2-CN, worin R2 die in Anspruch 1 angegebene Bedeutung hat, behandelt, um, nach einer Hydrolyse, ein Amid der Formel I, worin R1 für Wasserstoff steht, zu erhalten, welches man gewünschtenfalls in bekannter Weise mit einer Verbindung der Formel R1-I, worin R1 die in Anspruch 1 angegebene Bedeutung hat, in Gegenwart von Natriumhydrid alkyliert.

4. Verfahren zur Herstellung von Verbindungen der Formel I, wie sie in Anspruch 1 angegeben sind, dadurch gekennzeichnet, daß man eine Verbindung der Formel II

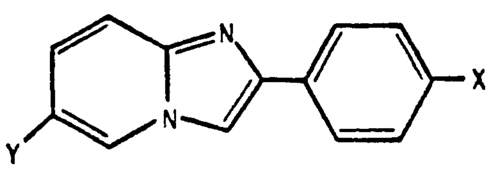

(II)

mit einem Amid der Formel

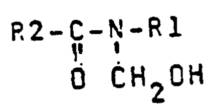

in welcher R1 und R2 die in Anspruch 1 angegebene Bedeutung haben, bei Raumtemperatur in Gegenwart von Schwefelsäure und gegebenenfalls mit einem Co-Lösungsmittel, wie Eisessig, reagieren läßt.

5. Verfahren zur Herstellung von Verbindungen der Formel I, wie sie in Anspruch 1 angegeben sind, dadurch gekennzeichnet, daß man eine Verbindung der Formel II

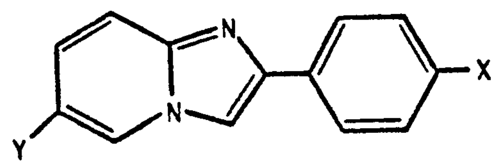

(II)

mit Paraformaldehyd und einem Nitril der Formel R2-CN, worin R2 die in Anspruch 1 angegebene Bedeutung hat, in der Hitze in essigsaurem Medium und in Gegenwart von Schwefelsäure reagieren läßt und nach einer Hydrolyse gewünschtenfalls die erhaltene Verbindung in bekannter Weise mit einer Verbindung der Formel R1-I, worin R1 die in Anspruch 1 angegebene Bedeutung hat, in Gegenwart von Natriumhydrid alkyliert.

6. Pharmazeutische Zusammensetzung, dadurch gekennzeichnet, daß sie eine Verbindung nach Anspruch 1 oder 2 in Kombination mit einem geeigneten Bindemittel enthalt.

**Patentansprüche** für den Vertragsstaat AT

1. Verfahren zur Herstellung von Verbindungen der allgemeinen Formel I

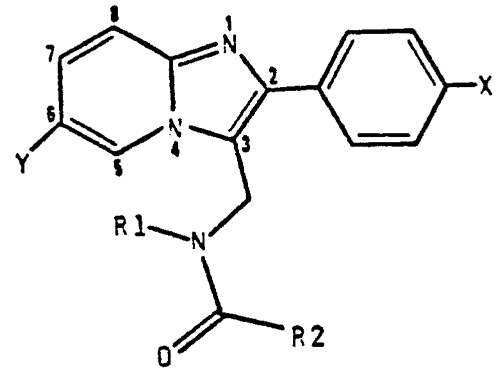

(I)

in welcher

X für Wasserstoff, ein Halogen oder eine $C_1$-$C_4$-Alkyl-, eine Methoxy, Methylthio-, Ethylthio-, Methylsulfonyl-, Nitro-, Amino-, Methylamino-, Dimethylamino-, Acetylamino- oder Diacetylaminogruppe,

Y für Wasserstoff, Chlor oder eine Methylgruppe,

R1 für Wasserstoff oder eine geradkettige oder verzweigte $C_1$-$C_4$-Alkylgruppe oder eine Benzylgruppe und

R2 für eine lineare oder verzweigte $C_1$-$C_6$-Alkylgruppe, eine Cyclohexyl-, Trichlormethyl-, Propen-1-yl-, Allyl-, Phenyl-, 4-Chlorphenyl oder eine Benzylgruppe steht oder aber

R1 und R2 gemeinsam eine aliphatische $C_3$-$C_5$-Kette bedeuten, welches Verfahren dadurch gekennzeichnet ist, daß man eine Formylierung einer Verbindung der Formel II

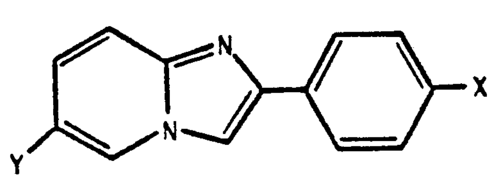

(II)

in welcher X und Y die oben genannte Bedeutung haben, mittels eines durch Einwirkung von Oxalylchlorid auf Dimethylformamid erhaltenen Reagens durchführt, um, nach einer Hydrolyse, einen Aldehyd der Formel III

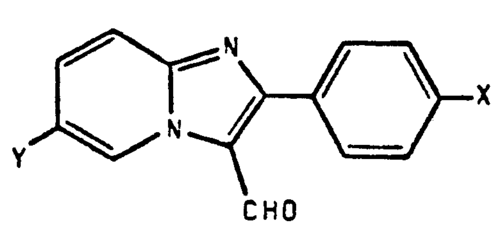

(III)

zu gewinnen, den man in bekannter Weise, vorzugsweise durch Einwirkung eines Alkalimetallborhydrids, zu einem Alkohol der Formel IV

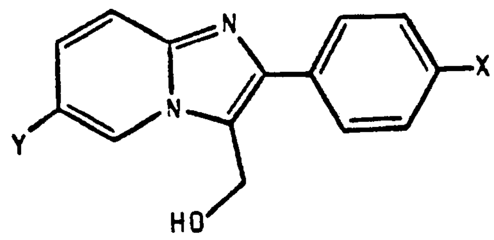

(IV)

reduziert, worauf man diesen letzteren in schwefelsaurem Medium mit einem Nitril der Formel R2-CN, worin R2 die oben genannte Bedeutung hat, behandelt, um, nach einer Hydrolyse ein Amid der Formel I, worin R1 Wasserstoff bedeutet, zu erhalten, welches man gewünschtenfalls in bekannter Weise mit einer Verbindung der Formel R1-I, in welcher R1 die oben genannte Bedeutung hat, in Gegenwart von Natriumhydrid alkyliert.

2. Verfahren zur Herstellung der Verbindungen der Formel I, wie sie in Anspruch 1 angegeben sind, dadurch gekennzeichnet, daß man eine Verbindung der Formel II

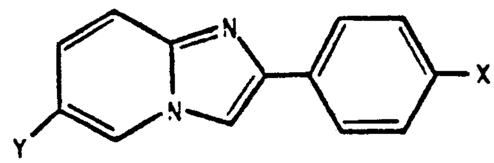

(II)

mit einem Amid der Formel

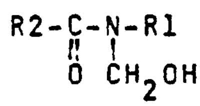

in welcher R1 und R2 die oben genannte Bedeutung haben, bei Raumtemperatur in Gegenwart von Schwefelsäure und gegebenenfalls mit einem Co-Lösungsmittel, wie Eisessig, reagieren läßt.

3. Verfahren zur Herstellung von Verbindungen der Formel I, wie sie in Anspruch 1 angegeben sind, dadurch gekennzeichnet, daß man ein Verbindung der Formel II

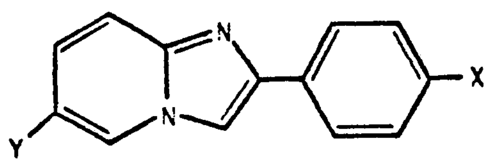

(II)

mit Paraformaldehyd und mit einem Nitril der Formel R2-CN, in welcher R2 die in Anspruch 1 genannte Bedeutung hat, in der Hitze in essigsaurem Medium und in Gegenwart von Schwefelsäure reagieren läßt und, nach einer Hydrolyse, gewünschtenfalls die erhaltene Verbindung in bekannter Weise mit einer Verbindung der Formel R1-I, in welcher R1 die in Anspruch 1 genannte Bedeutung hat, in Gegenwart von Natriumhydrid alkyliert.

**Claims** for the Contracting States: BE, CH, DE, FR, GB, IT, LI, LU, NL, SE

1. Compounds corresponding to the general formula I

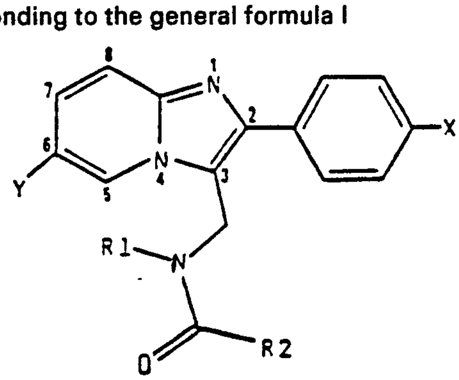

(I)

in which

X denotes hydrogen, a halogen or a $C_1$-$C_4$ alkyl group or a methoxy, methylthio, ethylthio, methylsulphonyl, nitro, amino, methylamino, dimethylamino, acetylamino or diacetylamino group,

Y denotes hydrogen, chlorine or a methyl group,

R1 denotes hydrogen or a linear or branched $C_1$-$C_4$ alkyl group, or a benzyl group,

R2 denotes a linear or branched $C_1$-$C_6$ alkyl group, or a cyclohexyl, trichloromethyl, 1-propenyl, allyl, phenyl, 4-chlorophenyl or benzyl group,

or alternatively

R1 and R2 together denote a $C_3$-$C_5$ aliphatic chain, as well as their addition salts with acids which are acceptable in pharmacology.

2. Compounds according to Claim 1, characterized in that X denotes chlorine or a methyl or methylthio group, Y denotes chlorine or a methyl group, R1 denotes hydrogen or a methyl group, and R2 denotes a propyl or isobutyl group.

3. Process for preparing the compounds of formula I as specified in Claim 1, characterized in that formylation is performed of a compound of formula II

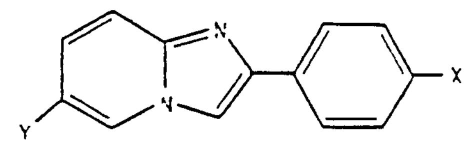

(II)

in which X and Y are as defined in Claim 1 by means of a reactant obtained by the reaction of oxalyl chloride with dimethyl-formamide, to obtain, after hydrolysis, an aldehyde of formula III

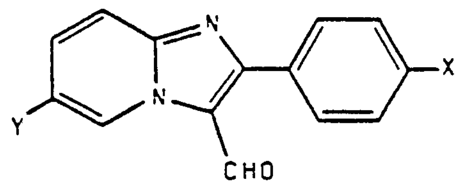

(III)

which is reduced in a known manner, preferably by reaction with an alkali metal borohydride, to an alcohol of formula IV

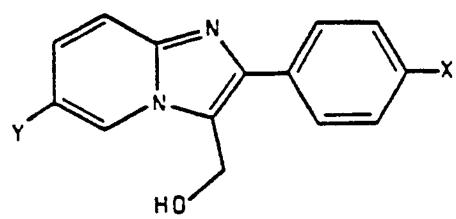

(IV)

and the latter is then treated in a sulphuric medium with a nitrile of formula R2-CN, in which R2 is as defined in Claim 1, to obtain, after hydrolysis, an amide of formula I in which R1 denotes hydrogen and, if so desired, this amide is alkylated in a known manner with a compound of formula R1-I, in which Ri is as defined in Claim 1, in the presence of sodium hydride.

4. Process for preparing the compounds of formula I as specified in Claim 1, characterized in that a compound of formula II

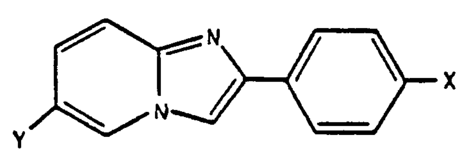

(II)

is reacted with an amide of the formula

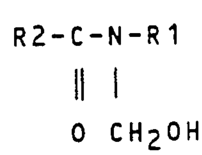

in which R1 and R2 are as defined in Claim 1, at room temperature in the presence of sulphuric acid, and optionally, with a cosolvent such as glacial acetic acid.

5. Process for preparing compounds of formula I as specified in Claim 1, characterized in that a compound of formula II

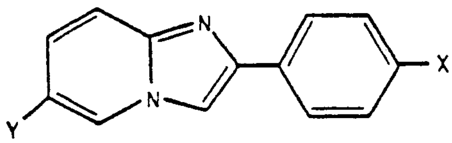

(II)

is reacted with paraformaldehyde and with a nitrile of formula R2-CN, in which R2 is as defined in Claim 1, with heating, in an acetic medium and in the presence of sulphuric acid and, after hydrolysis if so desired, the compound obtained is alkylated in a known manner with a compound of formula R1-I, in which R1 is as defined in Claim 1, in the presence of sodium hydride.

6. Pharmaceutical composition, characterized in that it contains a compound according to Claim 1 or 2, in combination with a suitable excipient.

**Claims** for the Contracting State: AT

1. Process for preparing compounds corresponding to the general formula I

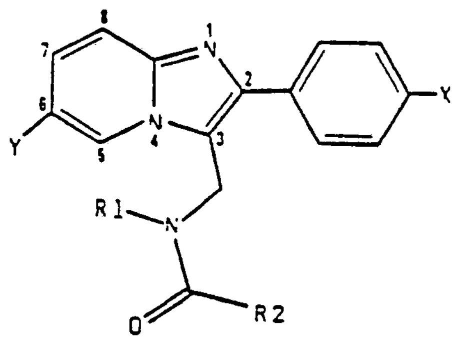

(I)

in which

X denotes hydrogen, a halogen or a $C_1$-$C_4$ alkyl group or a methoxy, methylthio, ethylthio, methylsulphonyl, nitro, amino, methylamino, dimethylamino, acetylamino or diacetylamino group,

Y denotes hydrogen, chlorine or a methyl group,

R1 denotes hydrogen or a linear or branched $C_1$-$C_4$ alkyl group, or a benzyl group,

R2 denotes a linear or branched $C_1$-$C_6$ alkyl group, or a cyclohexyl, trichloromethyl, 1-propenyl, allyl, phenyl, 4-chlorophenyl or benzyl group,

or alternatively

R1 and R2 together denote a $C_3$-$C_5$ aliphatic chain, which process is characterized in that a formylation of a compound of formula II

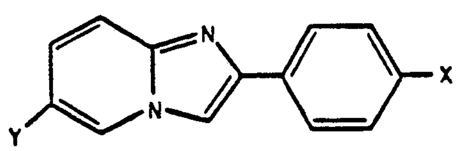

(II)

in which X and Y are as defined above, is performed by means of a reactant obtained by the reaction of oxalyl chloride with dimethylformamide, to obtain, after hydrolysis, an aldehyde of formula III

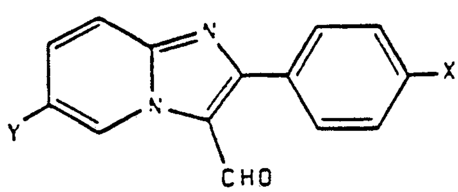

(III)

which is reduced in a known manner by reaction with an alkali metal boron hydride to an alcohol of formula IV

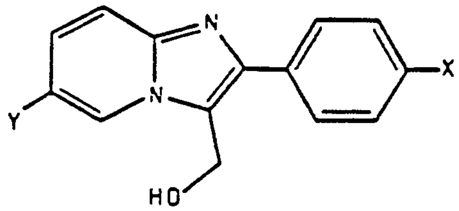

(IV)

and the latter is then treated in a sulphuric medium with a nitrile of formula R2-CN, in which R2 is as defined above, to obtain, after hydrolysis, an amide of formula I in which R1 denotes hydrogen and, if so desired, this amide is alkylated in a known manner with a compound of formula R1-I, in which R1 is as defined above, in the presence of sodium hydride.

2. Process for preparing the compounds of formula I as specified in Claim 1, characterized in that a compound of formula II

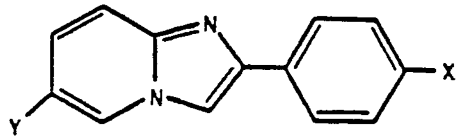

(II)

is reacted with an amide of the formula

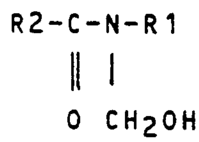

in which R1 and R2 are as defined in Claim 1, at room temperature in the presence of sulphuric acid and, optionally, with a cosolvent such as glacial acetic acid.

3. Process for preparing compounds of formula I as specified in Claim 1, characterized in that a compound of formula II

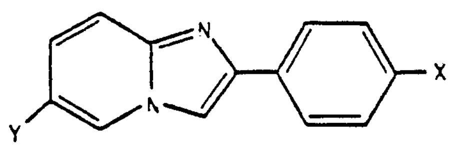

(II)

is reacted with paraformaldehyde and with a nitrile of formula R2-CN, in which R2 is as defined in Claim 1, with heating in an acetic medium and in the presence of sulphuric acid and, after hydrolysis, if so desired, the compound obtained is alkylated in a known manner, with a compound of formula R1-I, in which R1 is as defined in Claim 1, in the presence of sodium hydride.